# EUROPEAN PATENT APPLICATION

(11) **EP 4 850 747 A1**
(43) Date of publication of application: **09.12.2026**
(21) Application number: 26181957.7
(22) Date of filing: 29.05.2026
(51) Int. Cl.: A61N 1/05, A61N 1/36, A61N 1/372, A61N 1/08, A61B 5/055, A61B 5/00, A61N 1/37

(54) **SETTING OF MAGNETIC RESONANCE IMAGING MODE FOR AN IMPLANTED NEUROSTIMULATION DEVICE**

(30) Priority: 03.06.2025 US 202563816626 P; 01.07.2025 EP 25186539
(71) Applicant: BIOTRONIK SE & Co. KG, 12359 Berlin (DE)
(72) Inventor: Lim, Koeun, Tucson, 85750 (US); Kibler, Andrew B., Lake Oswego, 97035 (US); Slee, Sean, Tigard, 97224 (US); Qu, Min, Portland, 97223 (US)
(74) Representative: Biotronik Corporate Services SE

(57) **Abstract**

A computer-implemented method and associated computer program product for analyzing test data of measured impedances obtained from a neurostimulation device of the type that is magnetic resonance imaging (MRI) conditional and therefore has an MRI mode status of safe or unsafe for MRI. The test data form a time series of repeated lead impedance tests on the implanted neurostimulation device is referenced to an allowable range of impedances as defined by a device integrity specification to determine whether the test data are in-range or out-of-range. An MRI mode status for the device is set either to safe/enabled or unsafe/disabled based on this analysis. The MRI mode status is set to an enabled state, if a threshold plural number of test data sets have in-range lead impedance values, whereas the MRI mode status is set to a disabled state, if there is a recent test data set with an out-of-range lead impedance value.

## Description

The invention relates to neurostimulation devices and more specifically to a computer-implemented method and associated computer program product for setting the status of the magnetic resonance imaging (MRI) mode of an MRI-conditional neurostimulation device.

A significant proportion of patients implanted with a neurostimulation device, such as a spinal cord stimulation (SCS) device, will require access to MRI. The literature suggests that approximately 82% of SCS patients will need an MRI scan within 5 years after implantation [1]. While many SCS devices allow safe access to MRI, many are conditional on compliance with a system integrity specification which is not met by a significant fraction of the SCS patient population. It is reported that between 9-19% of all SCS device explants [2-4] are carried out to enable the patient to have an MRI scan. MRI compatibility varies widely across SCS devices but falls broadly into two categories: MRI-unsafe, meaning that it is not safe to carry out an MRI scan when the patient has an implanted SCS device, and MRI-conditional [5], meaning that MRI is safe with the implanted SCS device provided that the system integrity specification for the SCS device is met, wherein the system integrity specification specifies a number of conditional criteria. While many older SCS devices are MRI-unsafe, since they contain ferromagnetic metal, in recent years the technology has improved dramatically and most modem SCS devices are classified as MRI-conditional [6].

The conditional criteria of the system integrity specification for MRI-conditional SCS devices include safe ranges of certain MRI scan parameters (e.g. magnetic field strength, spatial magnetic field gradient) as well as SCS device integrity specifications, which include a requirement that the contact impedances of each of the implanted leads must be within a specified range [7]. By way of general background, an implanted neurostimulation device will have typically multiple leads that extend from the neurostimulation device to various points of a nerve or other tissue. Impedance can be measured, for example, as described in US 2012/038477 A1 [16] using an external testing device which is in data communication with the implanted SCS device. The present invention relates to MRI-conditional neurostimulation devices. An MRI-conditional neurostimulation device has a binary MRI mode status with the states enabled and disabled, which indicate whether it is safe or unsafe to carry out an MRI scan on a patient implanted with the neurostimulation device. The MRI mode status is set according to the results of testing the device against the MRI-conditional criteria.

An out-of-range lead impedance measurement is the most often cited cause for MRI-conditional failure in SCS devices and consequent setting of the MRI mode to disabled [5, 8, 9]. Contacts with impedance values above the specified range are considered to potentially result from lead fracture, with an occurrence rate of 5.9-9.1% of tested SCS devices [10, 11]. Contact impedance values below the specified range may indicate electrical short circuits. In either of these cases, system integrity is considered questionable, and the MRI mode status is set to a disabled state in order to ensure patient safety. Currently, in the United States, the practice is that when an out-of-range lead impedance measurement is detected, the MRI mode status is set to a disabled state indefinitely unless and until the MRI mode status is later reset to an enabled state by a clinician.

The upper and lower threshold impedance values that define the permissible lead impedance range are not defined by an industry standard. Rather, each SCS device manufacturer sets the permissible MRI-conditional impedance range, and each SCS device is accordingly labeled, as determined in the United States by the Food and Drug Administration (FDA) [12]. For example, the upper threshold impedance value for SCS devices manufactured by Nevro Corporation is 10 kOhm whereas the value for SCS devices manufactured by Boston Scientific Corporation is 4.5 kOhm [13, 14].

The literature provides some evidence that out-of-range lead impedance measurements are relatively common. A previous study presented a case series of three patients who needed MRIs but were barred due to their SCS devices having too-high electrode impedances [8].

Two of the three patients elected to have their SCS device explanted. Another retrospective study presented data from 327 SCS device patients who had a documented post-operative impedance check [9]. They found that 13/327 (4%) of patients had out-of-range lead impedances and would be ineligible for an MRI. Although this study provides valuable data, interpretation is hindered by the fact that the mean follow-up time was only 7.8 months postimplant. A more recent single-center retrospective study presented data from 363 patients with impedance-dependent MRI-conditional systems collected over a 5 year period [5]. This study had a longer mean follow-up time of 2.25 years and a Kaplan-Meier survival analysis was performed to map the survival of MR-conditionality over time. The study found that the risk of failure of lead impedances increased by 35.4% with each successive year to a peak of 43% of all leads by year 5. The mean overall survival time of normal lead impedances was 4.77 years.

Evidence reported in the literature demonstrates that many SCS patients implanted with MRI-conditional SCS devices are ineligible for MRI because the MRI mode status is set to a disabled state following an out-of-range impedance measurement.

Although sustained high lead impedances are often caused by lead fracture [10, 11], other transient patterns may stem from different causes. The literature suggests using a single measurement of the lead impedances results in a significant false positive rate for setting the MRI mode to disabled. For example, it has been noted in previous studies that lead migration can cause high impedance measurements [7, 8]. Transient low impedances have been reported in cases with closely spaced midline lead placements in which a low contact impedance is due to a nearby reference electrode on the other lead. A gradual increase in lead impedance of multiple leads connected to the implanted SCS device, sometimes to relatively high levels, is also observed to occur in the initial months post implant as scar tissue develops and encapsulation stabilizes before the lead impedance values then gradually reduce again. These examples demonstrate that out-of-range lead impedances are in some circumstances not indicative of lead fracture but have some other cause which need not result in the MRI mode being set to a disabled state.

After a potentially false positive that sets the MRI mode to disabled, accepted protocols permit the MRI mode to be reset to an enabled state by a clinician through a patient examination. This is a manual process carried out by an expert, which may not only take time for the expert to carry out but also take time to schedule.

US 2018/304087 A1 [14] issued as US 10279186 B2 provides for automated verification of the model type and lead type of an implanted neurostimulation device to ensure that MRI is not performed on an MRI-unsafe neurostimulation device but does not seek to address how to check whether an implanted MRI-conditional neurostimulation device currently meets its conditional criteria, i.e., does not check the MRI mode status of an MRI-conditional neurostimulation device.

US 2007/021814 A1 [15] issued as US 8140159 B2 discloses a method of performing dynamic impedance adjustment for an implantable medical device (IMD) that is implanted in a patient during an MRI scan to reduce energetic coupling between the applied magnetic field from the MRI system and the implanted IMD including its leads, e.g., when a resonance would otherwise cause dangerous levels of tissue heating.

According to one aspect of the disclosure, there is provided a computer-implemented method for determining a magnetic resonance imaging (MRI) mode status of an implanted MRI-conditional neurostimulation device, the method comprising:
providing a time series of test data sets containing measured lead impedance values, the time series extending over an observation window from a most recent test data set to a least recent test data set;
providing a device integrity specification defining an allowable range of impedance values for leads attached to the neurostimulation device;
comparing the test data sets against the device integrity specification to determine the number of test data sets that have in-range impedance values as defined by lying within the allowable range and as counted from the most recent test data set and working backwards in time through the time series; and
setting an MRI mode status to an enabled state, if the counted number of test data sets that have in-range impedance values is equal to or greater than a threshold number, wherein the threshold number is at least two, else setting the MRI mode status to a disabled state.

According to a further aspect of the disclosure there is provided a computer program product comprising a computer-readable storage medium having program code stored therein that is configured to allow a computer processor to perform the above-described method.

Requiring multiple in-range impedance measurements over a certain time window, e.g., several days, to set the MRI mode status to an enabled state is a safer approach than a clinician setting the MRI mode status to an enabled state based on a single in-range lead impedance measurement immediately prior to an MRI scan. For example, a single lead impedance measurement may represent a false negative, e.g., when the patient posture for a short time brings the ends of a fractured lead together, so that the MRI mode status is set to an enabled state even though an MRI scan would not be safe. Furthermore, a single lead impedance measurement may also represent a false positive, e.g., following the patient adopting a transient posture that strains the leads to temporarily separate the contact formed by the lead end in its socket in the SCS device header, in which case the MRI mode would be set to a disabled state unnecessarily.

The computer-implemented method for determining the MRI mode status may be carried out at any computing node of the MDCS, for example by the neuromodulation device itself, by the patient remote associated with the neuromodulation device, or by the neuro-service data center (including a portal in data communication with the neuro-service data center. The determined MRI mode status can then be distributed throughout the relevant nodes of the MDCS so that it is synchronized at the different levels of the MDCS, specifically synchronized between the neuromodulation device, the patient remote associated with the neuromodulation device, and the neuro-service data center.

Specifically, according to a still further aspect of the disclosure, there is provided a neurostimulation device comprising a computer-readable storage medium and a processor, the storage medium having program code stored therein and the processor being operable to execute the program code to perform the above-described method.

Moreover, according to a yet further aspect of the disclosure, there is provided a hand-held computing device comprising a computer-readable storage medium loaded with a therapy program, a processor and a wireless transceiver configured to communicate with a neurostimulation device, the storage medium having program code stored therein and the processor being operable to execute the therapy program and the program code to perform the above-described method.

Further, according to a yet still further aspect of the disclosure, there is provided a data center comprising a computer-readable storage medium, a processor and a communication transceiver to enable data communication within a medical device communication system, the storage medium having program code stored therein and the processor being operable to execute the program code to perform the above-described method.

After the MRI mode status has been set to a disabled state following an out-of-range impedance measurement, the above computer-implemented method provides an automated mechanism whereby the MRI mode can be reset to an enabled state without any manual intervention from an expert clinician and, moreover, in a way that does not compromise patient safety.

The time series may include test data sets obtained at regular time intervals, for instance at daily time intervals.

Comparing the data sets against the device integrity specification, includes, according to other embodiments, counting from the most recent test data set and working backwards in time through the time series and working backwards in time through the time series until a test data set with an out-of-range impedance value is encountered.

According to an embodiment, the counted number of test data sets that have in-range impedance values are appearing consecutively in the time series of test data sets.

In certain embodiments, the threshold number of test data sets is equal to the number of test data sets contained in the observation window. In such embodiments, the method may further comprise setting the MRI mode status to a disabled state, if at least one of the test data sets within the observation window has an out-of-range lead impedance value, as defined by lying outside the allowable impedance range.

In other embodiments, the threshold number of test data sets is lower than the number of test data sets contained in the observation window, thereby defining an enable window, the enable window being shorter than the observation window and extending backwards in time from the most recent test data set. In such embodiments, the method may further comprise setting the MRI mode status to a disabled state, if at least one of the test data sets within the enable window has an out-of-range lead impedance value, as defined by lying outside the allowable impedance range. Moreover, if there is a gap in the time series through absence of a test data set for a particular time interval, then the counted number is not incremented for the gap and the enable window is extended backwards in time by one time interval.

The size of the observation window, the size of the optional enable window, and also the allowable impedance ranges need not be fixed but in some embodiments may be variable, i.e., adjustable, responsive to device data and/or patient data. For example, the enable window can be adjusted responsive to an analysis of trends in the test data sets that occur over a time period greater than the observation window. Moreover, the observation window can be adjusted responsive to an analysis of trends in the test data sets that occur over a time period greater than the observation window. Further, the allowable impedance range is defined by upper and lower impedance values, at least one of which is adjustable responsive to an analysis of trends in the test data sets that occur over a time period greater than the observation window.

The current MRI mode status may be accessible to the patient via the patient remote, e.g., it may be shown on the display of the patient remote.

The continual automated setting of the MRI mode status may use the test data set that is collected daily by the neurostimulation device, the test data set including device data, which will include the MRI mode status, and patient data, which provides information on the patient's usage of the device and potentially further data on the patient, such as their activity log. This data set is typically uploaded via the patient remote to the neuro-service data center through the MDCS, e.g., on a daily basis.

The proposed approach of continual automated setting of the MRI mode status based on analysis of a time series of impedance measurements may provide one or more of the following advantages:
- Increase efficiency - when taking a single lead impedance measurement immediately before an MRI scan, the risk of spontaneous failure is reduced, since the continual collection, storage and analysis of impedance measurements will provide a more reliable picture of MRI safety of an MRI-conditional neurostimulation device, so that any patient whose implant has fractured leads is identified in advance of scheduling for MRI.
- Increase accuracy - there is a reduction of false positive detection of a lead fracture leading to MRI mode disablement, since an out-of-range impedance measurement taken on one day that is caused by a temporary patient posture, and not lead fracture, can be automatically corrected for by re-enabling the MRI mode, once the required number of consecutive in-range impedance measurements has been collected, stored and processed.
- Reduce MRI scan delays - a manual check by an expert clinician with the expertise and authorization to re-enable the MRI mode after it has been disabled by an out-of-range impedance measurement is dispensed with in many cases, since there is provided an automated mechanism for self-correction of the MRI mode status to enabled after an out-of-range lead impedance measurement. A manual assessment is only still required if there has been a recent out-of-range lead impedance measurement. The reduction in the number of manual assessments reduces the workload of the clinicians and reduces the number of patients that are rejected from having a scheduled MRI scan at the time of their appointment.

This invention will now be further described, by way of example only, with reference to the accompanying drawings.
- Figure 1: is a schematic diagram of a neurostimulation device, in the form of an SCS device, arranged in a medical device communication system (MDCS) with standard architecture in which a computing device, such as a patient remote, can control the neurostimulation device.
- Figures 2A and 2B: are schematic views from the front and back of a handheld touch screen computing device, such as a smartphone or tablet, which may function as the patient remote.
- Figure 2C: is a schematic block diagram of the functional components of the computing device of Figures 2A and Figure 2B.
- Figures 3A to 3F: are schematic representations of different example time series of lead impedance test results and their associated MRI mode status settings according to an embodiment of the invention.
- Figure 4: is a table showing specific example combinations of enable window length and observation window length in units of days.
- Figure 5: is a flow diagram showing steps of a method for performing the method according to an embodiment of the invention.

In the following detailed description, for purposes of explanation and not limitation, specific details are set forth in order to provide a better understanding of the present disclosure. It will be apparent to one skilled in the art that the present disclosure may be practiced in other embodiments that depart from these specific details.

In the following detailed description, the invention is described by way of example with reference to the neurostimulation device being an SCS device. It will be understood that the invention applies more generally to the genus of neurostimulation devices.

An SCS device is a type of IMD that is used for chronic pain therapy of a patient. An SCS device uses electrical current sent through electrodes on one or more leads implanted in the epidural space dorsal to the spinal cord. An IMD typically forms part of a MDCS that is configured to upload clinical data from the IMD on a continual basis via a MDCS to a remotely located data repository, which may be a data center. A daily upload of clinical data overnight is a common approach. In an SCS device, clinical data includes both patient data relating to physiological monitoring of a patient's health, for example as collected by sensors of the IMD, and device data related to status and operation of the IMD, for example data logging each time an SCS device is used and at what stimulation level in terms of electrical stimulation pulse width and pulse frequency as well as control signals issued to the SCS device to optimize delivery of pain relief. These clinical data can then be accessed by health care staff accessing the data center using suitably designed software applications. A treatment plan for a patient, a so-called therapy program, can then be decided upon based on analysis of these clinical data. The analysis may be expert analysis by a health care professional or computer-automated analysis with a software program, e.g., running on computing resource at a data center, or a combination of both.

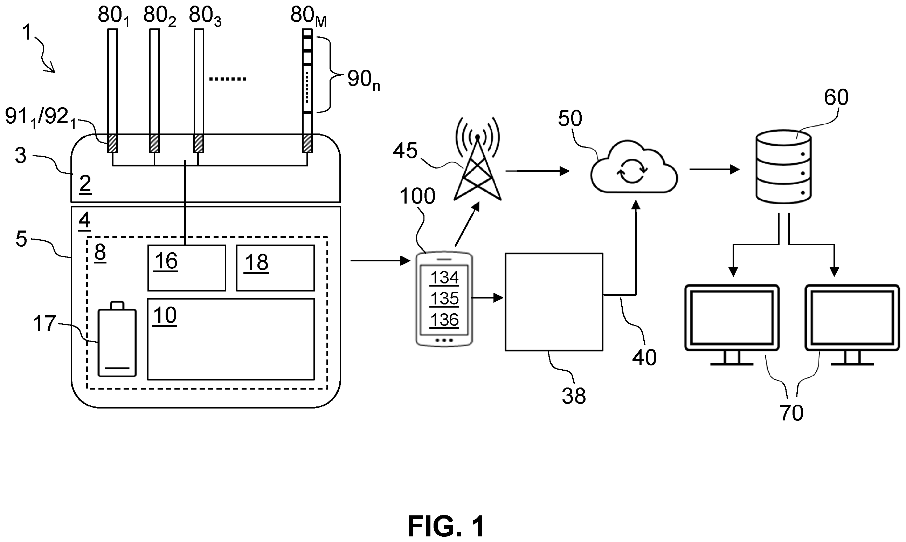

Figure 1 shows a MDCS of standard architecture which provides a communication platform between an IMD 1, such as an SCS device or other neurostimulation device, which is implanted in a patient and a remotely located data center 60, which is accessible to health care staff using suitable application software. The SCS device 1 comprises a header 2 and a can 4 which have respective outer casings 3 and 5. The can 4 includes therapeutic components for patient treatment, such as an SCS driver circuit 16.

The can 4 contains an electronics unit 8 including a system-on-a-chip (SoC) 10 with control electronic components for controlling the SCS device 1 and communication electronic components for external wireless communication. A rechargeable battery 17 provides electrical power to the electronics unit 8. The SoC 10 delivers control signals to the electrode driver circuit 16 according to a treatment plan devised by a computer program running on a processor within the SoC 10. The computer program is stored in a memory of the SoC 10. These hardware and software components operate collectively to provide an intelligent pulse generator to deliver electrical stimulation pulses to the electrodes conforming to a particular set of parameters, including amplitude, pulse width, frequency and/or duty cycle to provide stimulation therapy. For SCS, the electrodes are implanted at or near a patient's spinal cord to direct electrical signals into the patient's tissue for spinal cord stimulation. The SCS device 1 is implanted subcutaneously.

The header 2 is attached to a lead arrangement comprising *M* implantable percutaneous leads 80*ₘ* where m = 1 *to M* with each of the *m* leads incorporating *N* electrodes 90_{*m*,*n*} where *n =* 1 *to N.* The electrode driver circuit 16 provides a suitable drive current to each of the electrodes 90*_{m,n}* according to a set of control parameters that follow the treatment plan as controlled by the SoC 10. Each electrode 90_{*m*,*n*} is connected to an insulated wire (not shown) and the *n* insulated wires associated with any given one of the m leads are accommodated within a flexible insulated carrier which at one end terminates the lead 80*ₘ* in a lead end connector 91*ₘ*, which is configured as a complimentary connector to a connector port 92*ₘ*. A male-female relationship is shown between the lead end connector 91*ₘ* and the connector port 92*ₘ* respectively but this relationship may be reversed, or some other connector type may be used.

When the SCS device 1 is implanted, the rechargeable battery 17 can be charged in a contactless manner by an external charger (not shown), for example a resonant inductive charger, which is placed on the patient's skin adjacent the implanted can 4 to charge its rechargeable battery 17.

A hand-held patient remote 100 is present by which the SCS device 1 may be controlled. The computing device may be for the patient (i.e., a so-called "patient remote") or may be for a physician or field representative to perform equipment testing functions on the SCS device 1, e.g., as part of its initial set-up in a clinical setting. The hand-held patient remote 100 may, for example, be a smartphone or tablet with wireless transceivers 134, 135, 136. Wireless transceiver 134 is for wireless personal area network (WPAN) communication, such as Bluetooth Low Energy (BLE), Medical Implant Communication System (MICS) or Medical Device Radiocommunications Service (MedRadio). Wireless transceiver 135 is for cellular (e.g., 4G/LTE/5G) and wireless local area network (WLAN) communication. Wireless transceiver 136 is for wireless local area network (WLAN) communication.

If a smartphone is used, this is a smartphone that is possessed, e.g., owned, by the patient and has one or more software applications ('apps') installed on it for providing functionality relating to the SCS device 1, for example for control, testing and monitoring. There may for example be apps for the patient and apps for an expert to perform equipment testing functions. The apps are so-called Software as a Medical Device (SaMD) apps which are defined by the United States Food and Drug Administration (FDA) as software intended to be used for one or more medical purposes that perform these purposes without being part of a hardware medical device.

The cellular transceiver 135 and WLAN transceiver 136 provide two different data communication paths for the hand-held patient remote 100 to upload clinical data from the IMD 1 via an internet connection 50 to a remotely located data center 60 (hereinafter also called "backend 60") acting as repository for storage of clinical data and/or as a host for the services, for example a neuro-service data center. The remote's WLAN transceiver 136 can upload data to the backend 60 via a router 38 and a telephone line 40 (or telephone network 40) using a wired internet connection 50. The internet connection 50 may provide access to one or more distributed networks and/or cloud services. The remote's cellular transceiver 135 can upload data to the backend 60 via one or more cellular network base stations 45 (cellular towers), for example a low-power wide-area network (LPWAN)-capable cellular network base station. In some cases, instead of a public communication network, a dedicated point-to-point transmission, e.g., via a dedicated telephone line, may be provided for uploading clinical data. In all these scenarios, uploading of clinical data from the SCS device 1 to the backend 60 takes place via the intermediary of the hand-held patient remote 100, the latter thereby acting as a relay device.

Health care staff, such as health care professionals (HCPs), clinical specialists and representatives and remote care team members have access to the backend 60 via suitable portals 70 with the aid of a software application running on the backend 60 and/or the portal 70 to provide the necessary user interfacing, diagnostics and so forth. At least one portal 70 is provided by at least one workstation for health care staff to access a data center, e.g., the backend 60. Analysis and diagnostic software may also be run at the backend 60 to analyze clinical data from individual patients or groups of patients.

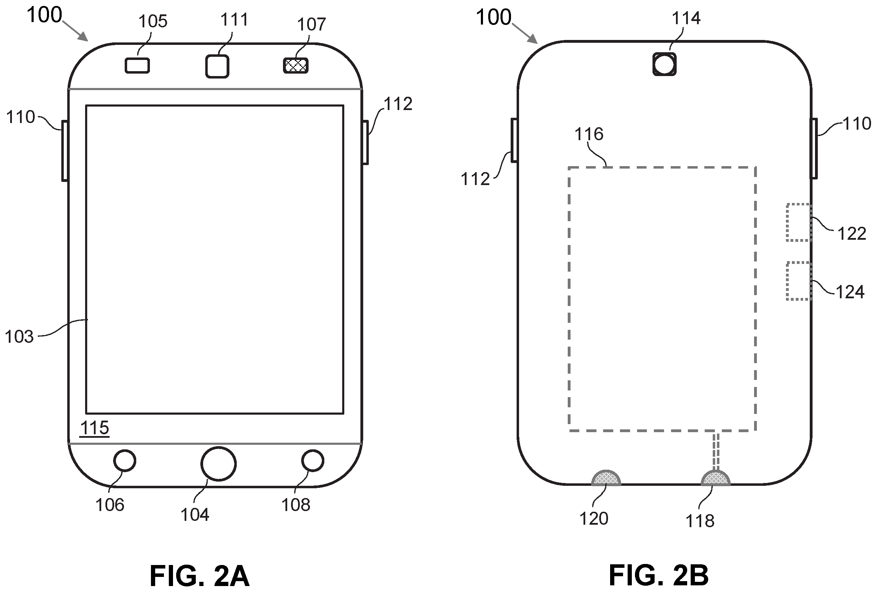

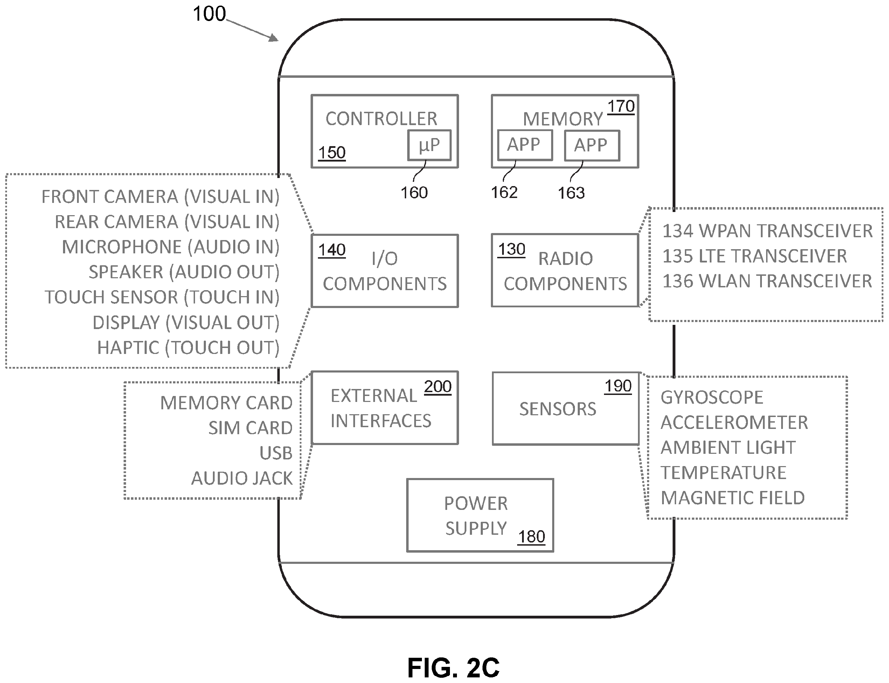

Figures 2A and 2B are schematic perspective views from the front and back of a handheld touch screen computing device such as a smartphone or tablet which may function as the patient remote 100. Figure 2C is a block diagram of the functional components of the computing device of Figures 2A and Figure 2B.

Referring to Figure 2A, the patient remote 100 has a smartphone or tablet format. The patient remote 100 is arranged in a housing with a front face (facing outwards from Figure 2A), a rear face and a bezel forming the edges of a substantially rectilinear object. The front face is mostly taken up with a touch screen display which combines a display 115 with a touch sensitive area 103. The touch screen enables the user to input commands to applications running on the computing device through gestures. The front face also accommodates a mechanical key (or button) 104 and two touch sensor keys (or buttons) 106, 108, one either side of the mechanical key 104. The edges of the housing accommodate a mechanical rocker switch 110 for volume control and an on/off switch 112.

A front facing camera 111 for capturing stills or video images is arranged on the front face near the top of the housing facing forwards and has adjacent to it a microphone 105 for capturing audio and a speaker 107 for outputting audio.

Referring to Figure 2B, the rear view, a rear facing camera 114 for capturing stills or video images is arranged near the top of the housing facing backwards. A battery 116 is accommodated within the housing and constitutes a power supply (shown with dashed lines). The power supply further includes an external power input socket 118 which may be used for powering the device as well as charging the battery. Alongside the power input socket 118 at the bottom of the device there is another external connector in the form of an audio jack 120 for audio output. Further external interfaces may be provided including various ports, holders, and sockets for physical connections. With dotted lines we show two internal holders 122, 124 which may be for a SIM card and a memory card or further SIM card. The memory card is a kind of data storage device.

Referring to Figure 2C, this shows selected functional components of the patient remote 100. The patient remote 100 has radio components 130, input/output (I/O) components 140, one or more controllers 150 associated with one or more processors 160 and one or more memories 170, a power supply 180, sensor components 190 and external interfaces 200. The memory 170 is operable to store 'apps', in particular a therapy programming app 162 for formulating and controlling delivery of a therapy program as well as any number of other miscellaneous apps 163, including for example other SaMD apps for monitoring and testing an IMD. Each app comprises software code portions that are loadable into and executable by the processor 160. The controller(s) 150 may include a touch sensor controller and a display controller, or a combined touch and display controller.

The processor(s) may comprise separate processing units for specialist tasks such as touch sensing, display drive, video processing, speech/audio analysis and/or speech/audio synthesis. The controller(s) and associated processor(s) and memory(ies) have the task of controlling the computing device and executing computer programs stored in the memory(ies). The memory(ies) may store computer applications for running on the computing device as well as collecting data from the various I/O devices. The controller(s) typically functions to control overall operation of the computing device, in addition to the operations associated with the 'apps'. The controller(s) processes signals, data, information and the like input or output through the above-mentioned components and/or runs application programs saved in the memory, thereby processing or providing a user with appropriate information and/or functions.

The I/O components 140 include a display capable of displaying content and also acting as part of a graphical user interface, wherein the display may be based on a suitable technology such as liquid crystal or organic light emitting diodes, as well as a position-sensitive touch sensor area overlaid on, or formed as an integral part of, the display to serve as part of a graphical user interface in conjunction with the display with optionally other touch sensor areas or buttons (e.g. on the reverse side or edge (bezel) of the device housing. Further I/O components, as previously mentioned, are front and rear facing cameras for capturing stills or video images, a microphone for capturing audio, a speaker for outputting audio and a haptic output embedded in the touch screen to provide tactile feedback. The sensing components include a gyroscope, an accelerometer, an ambient light sensor and a temperature sensor, for example. The external interfaces may include various ports and sockets for physical connections, such as a SIM card, wired LAN connectors, memory cards, audio jack socket, USB ports and so forth.

Having now described the general system environment and the relevant devices for the invention, the specifics of the present invention are described below.

The basic idea exploited by the present invention is that an MRI mode status program is provided which has the function of continually updating the MRI mode status based on a time series of recently collected lead impedance measurement test data from an implanted SCS device or other implanted neurostimulation device. For example, the lead impedance measurement test data may be extracted from the device data that is typically collected on at least a daily basis in modern SCS devices and then passed via the patient remote over the MDCS to the neuro-service data center. The MRI mode status setting program may, for example, be included as a component of the therapy program app 162 or as a component of another SaMD app 163, or as stand-alone SaMD app 163.

An MRI mode status for the device is set either to safe/enabled or unsafe/disabled based on an analysis of the time series of test data, more specifically based on referencing each test data set of the time series against an allowable range of impedances as defined by a device integrity specification, thereby to determine whether each test data set consists of only in-range lead impedances or includes at least one out-of-range lead impedance.

The MRI mode status is set to an enabled state, i.e., safe for MRI, when there have been a sufficient number of consecutive in-range lead impedance measurements over a certain time window without an out-of-range lead impedance measurement. An out-of-range lead impedance measurement results in the MRI mode being set to a disabled state. The time series that is included in the analysis is limited to the most recent test data sets, which we refer to as the observation window.

In some embodiments, all test data sets in the observation window must be in-range in order that the MRI mode status is set to an enabled state.

In other embodiments, this condition is relaxed to permit the setting of the MRI mode status to enabled when only a most recent subset of the test data sets are in-range and any out-of-range data set or sets within the observation window are sufficiently old. The number of consecutive in-range data sets that are required for setting the MRI mode status to enabled defines a time window, which we refer to as an enable window. The observation window is then somewhat longer than the enable window. The enable window length will typically be at least 60% of the observation window length, e.g. 3 out of 5 days. This approach can take account of the potential for gaps in the time series, e.g., caused by the neurostimulation device being fully discharged and not timely recharged by the patient. In this case, there will be missing data, e.g. from a particular day or days. The analysis allows for missing data by defining the condition for setting the MRI mode status to enabled such that gaps are effectively skipped, and the counting of consecutive in-range data sets is expanded to embrace a larger time window by moving further back in time by one element of the time series (but not beyond the end of the observation window). With an observation window of length 'm' and an enable window of length 'n' where *m* ≥ *n,* the MRI mode status is set to an enabled state if the *n* most recent lead impedance measurements that are within the observation window are in-range, else is set to a disabled state, i.e., unsafe for MRI. This means, for instance, that if any out-of-range lead impedance measurement has been detected within the *n* most recent lead impedance measurements, then the MRI mode status is set to a disabled state. It also means that if the number of days *k* of missing data within the observation window is too high, specifically *k* > (*m* - *n*)*,* then the MRI mode status is set to a disabled state. Generally, it will be advantageous to have an observation window that is somewhat longer than the enable window, for example 1, 2 or 3 days longer, in order to account for days where data is missing, for example due to the SCS device battery or the patient remote battery being fully discharged. One specific example combination that is explored in more detail further below is an observation window of 7 days paired with an enable window of 5 days. Another example combinations would be 6 and 4 days, 6 and 3 days, and 5 and 3 days for the observation and enable windows respectively. It is also possible in certain embodiments to have the observation window equal to the enable window; this will just mean that the MRI mode will be set to a disabled state whenever there is a day with missing data within the observation window.

The MRI mode status setting program may be stored in memory of the neurostimulation device, i.e., local to where the test data from the lead impedance testing is collected and executed by a processor of the neurostimulation device. Alternatively, the MRI mode status setting program may be stored in memory of the patient remote, or memory of the neuro data center, and executed by a processor of the patient remote or neuro data center respectively.

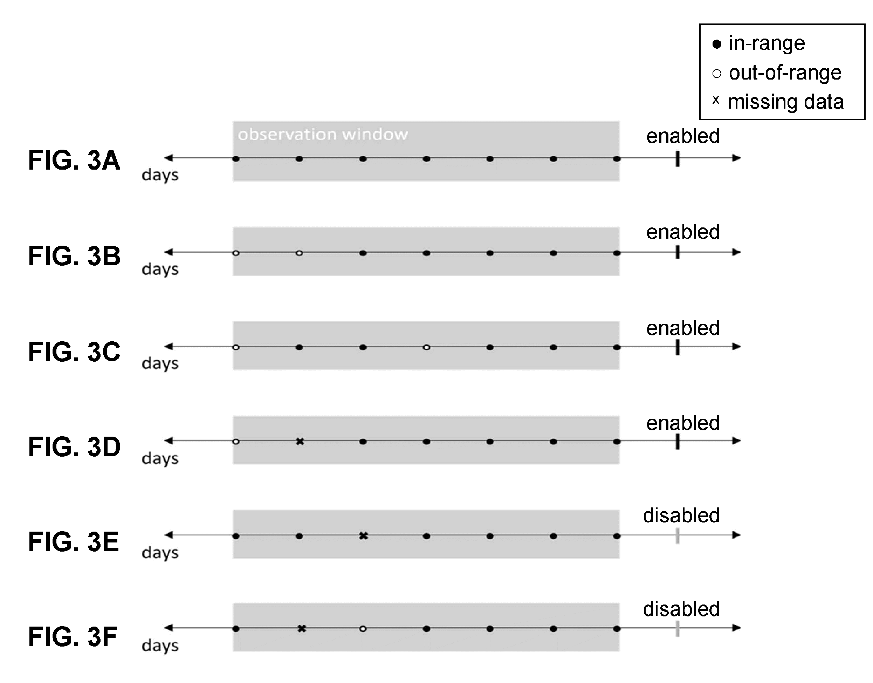

Figures 3A to 3F are schematic representations of 6 different example time series of lead impedance test results and their associated MRI mode status settings according to an embodiment of the invention. In each of these examples, an observation window of 7 days is used in combination with an enable window of 5 days. On the illustrated timelines left-to-right depicts the passage of time from the past to the present. The symbol key is shown inset for all of Figures 3A to 3F, wherein a solid circle indicates an in-range impedance measurement, a hollow circle indicates an out-of-range impedance measurement, and a cross indicates that there is missing data, i.e., on that day no device data was obtained. A reason for this might be that the SCS device battery was fully discharged, and the patient did not recharge it for some time.

The first example shown in Figure 3A is when all lead impedance measurements within the 7-day observation window are in range, so the MRI mode status is set to an enabled state.

The second example shown in Figure 3B has missing data for the two oldest days followed by five consecutive days of in range measurements, so the MRI mode status is set to an enabled state, since the enable window width is 5 days.

The third example shown in Figure 3C has missing data on today-minus-three and today-minus-six but in-range measurements on the other days within the observation window, so the MRI mode status is set to an enabled state, since the most recent 5 days of available data (skipping the missing data on today-minus-three and taking account of the in-range measurements on today-minus-four and today-minus-five) have in-range measurements.

The fourth example shown in Figure 3D has missing data on today minus six, an out-of-range impedance measurement on today-minus-five and in-range measurements on the other days within the observation window. Since the enable window is 5 days and the five most recent measurements are in range, the MRI mode status is set to an enabled state.

The fifth example shown in Figure 3E has no missing data but an out-of-range impedance measurement on today-minus-four. Since today-minus-four is within the 5 day enable window, then the MRI mode status is set to a disabled state. In a continuation of the data pattern of the fourth example, if the next day yielded an in-range measurement, then the MRI mode status would switch to enabled, since then the 5-day enable window would cover only the last 5 days, which all would have in range measurements. On the other hand, in an alternative continuation of the data pattern of the fourth example, if the next day failed to provide data, i.e., missing data, then the 5-day enable window would encompass the day with the out-of-range impedance measurement, so the MRI mode status would be disabled.

The sixth example shown in Figure 3F has missing data on today-minus-four, an out-of-range impedance measurement on today-minus-five and in-range measurements on the other days within the observation window. Therefore, on account of the day with the missing data, the 5-day enable window would encompass the day with the out-of-range impedance measurement, and the MRI mode status would be set to a disabled state.

It is noted that, if there is missing data on three or more days, then the MRI mode would inevitably be set to a disabled state, since there would then only be four days with data within the observation window, so even if all four of these days returned in-range measurements that would not reach the threshold of 5 days specified for the enable window in these examples.

Furthermore, if there is insufficient lead impedance measurement data the MRI mode status may be set to a disabled state by default. For example, the MRI mode status can be set to a disabled state at the time of implant, and once an amount of time corresponding to at least the enable window has elapsed the MRI mode status will change to enabled following the time series building up to fill the enable window and observation window and there having been a sufficient number of consecutive in-range lead impedance measurements. Alternatively, the MRI mode status could be set to an enabled state at the time of implant, if the clinician has carried out a lead impedance test and is satisfied of the integrity of the lead connections to the can, in which case it will require an out-of-range test measurement before the MRI mode status changes to disabled.

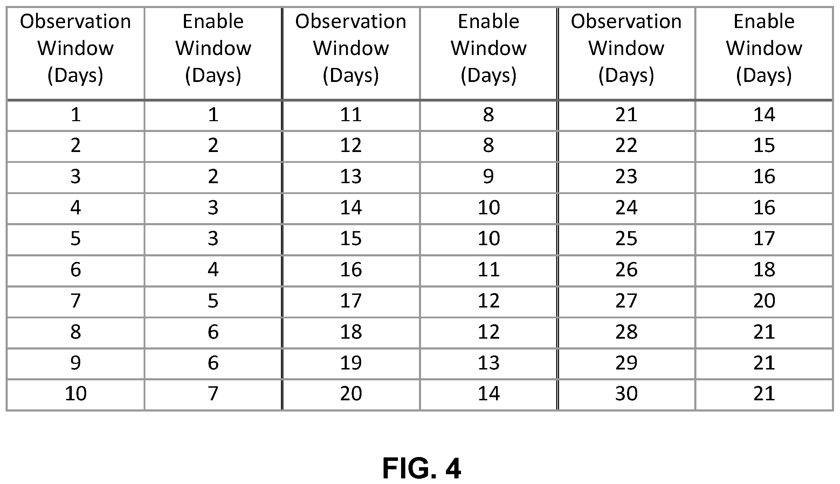

Figure 4 is a table showing by way of example only specific example combinations of enable window length and observation window length in units of days. These work through the permutations of an observation window of between 1 and 30 days paired with an enable window of between 1 and 21 days. In alternative examples, the units could be different, e.g., a fraction of a day such as 12 hours (half a day) or multiple days such as 7 days (a week). Moreover, the time interval between measurements need not be equal, as for example would be the case when measurement was triggered asynchronously, such as at each therapeutic use of the neurostimulation device by a patient. Data collection could combine these timings, so for example the data set may include both daily data and use-triggered data.

A refinement may be to require not only that a certain number of consecutive in-range lead impedance measurements are observed but also that they have a certain time distribution that prevents the MRI mode being set to an enabled state based on the threshold number of in-range impedance measurements occurring in time too close together, e.g., over a period that is much shorter than a day, so may only capture the patient when in a single posture (e.g. sitting or sleeping) or performing a single activity type (e.g. walking).

The lead impedance measurement data is preferably shared and synchronized between relevant nodes of the MDCS, in particular between the neuro data center, the patient remote, and the neurostimulation device. The data sharing and synchronization should cover at least the data relating to the current observation window. The lead impedance measurement data can thus be synchronized at each node of the MDCS as desired. The synchronization period should preferably at least cover the observation window but may include a longer period, such as a month or multiple months, or in some cases the entire time window from implantation. Alternatively, the full historical data set may only be stored at the neuro data center, and optionally the patient remote, but not at the neurostimulation device. Synchronization may be maintained at all times or performed at intervals, for example once a day, or whenever a new lead impedance test is carried out at the neurostimulation device. In the case that the lead impedance measurement data from the day before (previous night's measurement) is missing, i.e., not available, or in the case that the number of impedance measurements stored is less than the enable window, then synchronization may be attempted by the patient remote at the time of an MRI mode entry attempt.

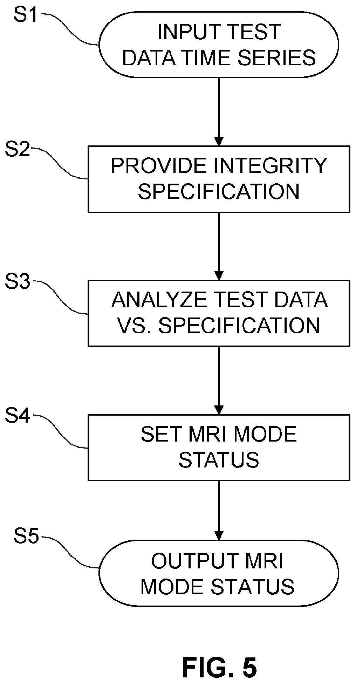

Figure 5 is a flow diagram showing steps of a method for performing the method according to an embodiment of the invention.
- In Step S1, a time series is input that comprises multiple test data sets forming a time series, each test data set containing lead impedance values from a test performed at a particular time. The time series extends in time from the newest, i.e., most recently collected, test data set through to the oldest, i.e., least recent, test data set. The time series is finite, terminating at the 'old' end of a defined observation window. The observation window may be defined by a time span or by a number of data sets or a combination of both such as at least a certain number of data sets that span at least a certain minimum amount of time. The time series may consist of or include test data sets obtained at regular time intervals, such as daily intervals.
- In Step S2, a device integrity specification is made available to the method, the device integrity specification defining an allowable impedance value range for the leads that are attached to the implanted neurostimulation device. These are the leads that have the function of delivering electrical stimulation pulses and which are surgically embedded in tissue at one end and attached to the can of the neurostimulation device by the other end.
- In Step S3, the test data sets are benchmarked against the device integrity specification in respect of lead impedance. It is determined whether for each test data set, starting from the most recent one and working back in time, whether or not the test data set has in-range or out-of-range lead impedance values. A number is then obtained for how many in-range test data sets there are counting back from the most recent one. To account for possible gaps in the time series, an enable window may be defined, like the observation window, to extend to the most recent test data set but is somewhat shorter than the observation window. The enable window provides additional functionality when the threshold plural number of test data sets is lower than the number of test data sets contained in the observation window. In that case, when a gap in the time series is encountered, i.e., a data set is missing, the number of in-range test data sets is not incremented but the enable window is extended backwards in time by one time interval provided that the old end of the observation window has not been reached.
- In Step S4, the MRI mode status is set. The MRI mode status is set to an enabled state if the number of test data sets with in-range lead impedance values, as counted according to the procedure of Step S3, is equal to or greater than the threshold value. The threshold value is an integer number of at least two. More commonly, the threshold value will be set to at least three, with values of four, five, six and seven being further specific examples that may be used. Moreover, in embodiments without a shorter enable window, the MRI mode status is set to a disabled state, if at least one of the test data sets within the observation window has out-of-range lead impedance values, whereas, in embodiments with an enable window shorter than the observation window, the MRI mode status is set to a disabled state, if at least one of the test data sets within the enable window has out-of-range lead impedance values.
- In Step S5, the MRI mode status as set in Step S4 is output.

Various additional design options of the proposed approach are now discussed.

The data set used by the MRI mode status setting program may include additional data that forms part of, or is derived from, the patient and device data collected by the neurostimulation device and/or by the patient remote, such as additional data relating to device status and patient usage of the neurostimulation device. An example of such additional data would be data relating to physiological status of a patient or physical status of the device is to take account of trends in the lead impedance measurement values over a timescale that is longer than the observation window. Specifically, a physiological status inference can be inferred from the spatial and temporal distribution of lead impedance measurement values. Namely, a gradual increase in lead impedance that is correlated over multiple leads connected to the implanted SCS device in the weeks after device implantation may indicate post-operative tissue inflammation or tissue scarring (and not lead fracture). If such a trend is identified and the lead impedance values approach the upper threshold, then the MRI mode status setting program may be configured to increase the upper threshold temporarily by up to a certain amount, and for a limited amount of time, in the expectation that the measured impedance values will reduce again after a few weeks. Should this reduction not occur within a certain time, then the MRI mode status is then set to a disabled state, and the upper threshold is reset to its original value.

The MRI mode status setting program may determine on an ongoing basis a probability that the leads are functional and a probability that there is a permanent failure of the leads.

The MRI mode status setting program may not only output the MRI mode status but may additionally be configured to provide a further output in certain circumstances that is a trigger for further action. The further output may be acted on by the neurostimulation device and/or trigger an action by the patient remote or at the neuro data center. For example, multiple consecutive out-of-range impedance measurements, out-of-range impedance measurements that are out-of-range low or out-of-range high, or there are out-of-range impedance measurement on multiple, or all, leads of the neurostimulation device may be used to generate hardware triggers or warning messages or flags according to more complex logic.

The further output may be acted upon by the neurostimulation device to:
- shut down therapy delivery,
- selectively deactivate transmission of stimulation pulses to any lead with an out-of-range impedance, and/or
- increase the frequency of lead impedance measurements beyond the regular (e.g. daily) schedule so there is a more extensive data set to provide input to the MRI mode status setting program and also to any clinician who reviews the data.

The further output may be acted upon by the patient remote to generate a message to inform and prompt action by the patient, such as to notify the patient that therapy delivery has been shut down and to seek a consultation.

The further output may set a warning flag at the neuro data center to prompt a remote care team member who is responsible for patient monitoring to review the data set relating to device status and patient usage of the device. The further output to the neuro data center may be triggered when the MRI mode status is set to a disabled state for longer than a certain period, for example longer than a certain integer or non-integer multiple of the observation window or enable window, or after a certain threshold number of consecutive out-of-range impedance measurements. This would then prompt a remote care team member to review the device data set.

The MRI mode status setting program may be configured to dynamically adjust the observation window and/or the enable window based on longer term trends in the impedance measurement values, i.e., historical data, and/or correlation between impedance values of multiple leads of the neurostimulation device taken at the same time. For example, when the historical data for the neurostimulation device shows consistently stable in-range lead impedance measurements over an extended period, e.g., several months, the enable window could be shortened, so that the MRI mode status is set to an enabled state more quickly after an out-of-range lead impedance measurement. The enable window would then be increased again if appropriate in response to any adverse trend in the lead impedance measurements.

In summary of the above, a computer-implemented method and associated computer program product is provided for analyzing test data of measured impedances obtained from a neurostimulation device of the type that is MRI-conditional and therefore has an MRI mode status of safe or unsafe for MRI. The test data form a time series of repeated lead impedance tests on the implanted neurostimulation device is referenced to an allowable range of impedances as defined by a device integrity specification to determine whether the test data are in-range or out-of-range. An MRI mode status for the device is set either to safe/enabled or unsafe/disabled based on this analysis. The MRI mode status is set to an enabled state, if a threshold plural number of test data sets have in-range lead impedance values, whereas the MRI mode status is set to a disabled state, if there is a recent test data set with an out-of-range lead impedance value.

### REFERENCES

1. Desai MJ, Hargens LM, Breitenfeldt MD, et al. The rate of magnetic resonance imaging in patients with spinal cord stimulation. Spine (Phila Pa 1976). May 1 2015;40(9):E531-537.
2. Al-Kaisy A, Royds J, Al-Kaisy O, et al. A Review of Literature and Case Series. Reg Anesth Pain Med. Nov 2020;45(11):883-890.
3. Moeschler SM, Sanders RA, Hooten WM, Hoelzer BC. Spinal cord stimulator explantation for magnetic resonance imaging: a case series. Neuromodulation. Jun 2015;18(4):285-288; discussion 288.
4. Pope JE, Deer TR, Falowski S, et al. Multicenter Retrospective Study of Neurostimulation With Exit of Therapy by Explant. Neuromodulation. Aug 2017;20(6):543-552.
5. Mullins CF, Harris S, Pang D. A retrospective review of elevated lead impedances in impedance-dependent magnetic resonance-conditional spinal cord stimulation devices. Pain Practice. 2023;24(2):270-277.
6. Sayed D, Chakravarthy K, Amirdelfan K, et al. A Comprehensive Practice Guideline for Magnetic Resonance Imaging Compatibility in Implanted Neuromodulation Devices. Neuromodulation. Oct 2020;23(7):893-911.
7. Fisher KA, Jenkins TC, Singh V, Li R, Qadri YJ. MR-conditionality failure modes: a comparison across various spinal cord stimulators. Pain Manag. Sep 13 2024:1-5.
8. Jotwani R, Abd-Elsayed A, Villegas K, et al. Failure of SCS MR-Conditional Modes Due to High Impedance: A Review of Literature and Case Series. Pain Ther. Jun 2021;10(1):729-737.
9. Hagedorn JM, Parmele JB, Wolff JS, Bendel MA, D'Souza RS. The Prevalence of Elevated Impedances and Magnetic Resonance Imaging Ineligibility Following Implantation of 10 kHz Spinal Cord Stimulation Devices: A Retrospective Review. Neuromodulation. Jul 2022;25(5):719-723.
10. Cameron T. Safety and efficacy of spinal cord stimulation for the treatment of chronic pain: a 20-year literature review. J Neurosurg. Mar 2004;100(3 Suppl Spine):254-267.
11. Deer TR, Mekhail N, Provenzano D, et al. The appropriate use of neurostimulation: avoidance and treatment of complications of neurostimulation therapies for the treatment of chronic pain. Neuromodulation Appropriateness Consensus Committee. Neuromodulation. Aug 2014;17(6):571-597; discussion 597-578.
12. Chandrasekaran S, Nanivadekar AC, McKernan G, et al. Sensory restoration by epidural stimulation of the lateral spinal cord in upper-limb amputees. Elife. Jul 21 2020;9.
13. Nevro Corporation "1.5 Tesla and 3 Tesla Magnetic Resonance Imaging (MRI) Guidelines for the SENZA Neuromodulation Systems" February 2024.14. Boston Scientific Corporation "ImageReady MRI Guidelines for Precision Spectra Spinal Cord Stimulator System" 2019.
14. US 2018/304087 A1
15. US 2007/021814 A1
16. US 2012/038477 A1

### REFERENCE NUMERALS

- 1: neurostimulation device, e.g., SCS device
- 2: header
- 3: header outer casing
- 4: can
- 5: can outer casing
- 8: electronics unit
- 10: system-on-a-chip (SoC)
- 16: driver circuit
- 17: neurostimulation device battery
- 18: test circuit
- 38: router
- 40: telephone line / telephone network
- 45: cellular network base station (cellular tower)
- 50: internet connection
- 60: remotely located data center (backend)
- 70: portal
- 80: leads
- 90: electrode
- 91: lead end connector
- 92: connector port
- 100: patient remote, e.g., smartphone or tablet handheld computing device
- 103: touch sensitive area
- 104: mechanical key (or button)
- 105: microphone (audio in)
- 106: touch sensor key (or button), first
- 107: loudspeaker (audio out)
- 108: touch sensor key (or button), second
- 110: mechanical rocker switch for volume control
- 111: front facing camera
- 112: on/off switch
- 114: rear facing camera
- 115: display
- 116: battery
- 118: external power input socket
- 120: audio jack (audio out)
- 122: internal card holder, first
- 124: internal card holder, second
- 130: radio components
- 134: smartphone local transceiver (e.g. WPAN such as BLE)
- 135: smartphone cellular transceiver (e.g., 4G/LTE/5G)
- 136: smartphone wireless local area network (WLAN) transceiver
- 140: input/output (I/O) components
- 150: controller(s)
- 160: processor(s)
- 162: therapy programming app
- 163: miscellaneous app
- 170: memory(ies)
- 180: power supply

## Claims

1. A computer-implemented method for determining a magnetic resonance imaging (MRI) mode status of an implanted MRI-conditional neurostimulation device, the method comprising:
providing a time series of test data sets containing measured lead impedance values, the time series extending over an observation window from a most recent test data set to a least recent test data set (S1);
providing a device integrity specification defining an allowable range of impedance values for leads attached to the neurostimulation device (S2);
comparing the test data sets against the device integrity specification to determine the number of test data sets that have in-range impedance values as defined by lying within the allowable impedance range and as counted from the most recent test data set and working backwards in time through the time series; and
setting an MRI mode status to an enabled state, if the counted number of test data sets that have in-range impedance values is equal to or greater than a threshold number, wherein the threshold number is at least two, else setting the MRI mode status to a disabled state (S4).

2. The method of claim 1, further comprising the step of: for comparing the data sets against the device integrity specification, counting from the most recent test data set and working backwards in time through the time series until a test data set with an out-of-range impedance value is encountered (S3).

3. The method of claim 1 or 2, wherein the counted number of test data sets that have in-range impedance values are appearing consecutively in the time series of test data sets.

4. The method of at least one of the preceding claims, wherein the threshold number of test data sets is equal to the number of test data sets contained in the observation window.

5. The method of at least one of the preceding claims, further comprising setting the MRI mode status to a disabled state, if at least one of the test data sets within the observation window has an out-of-range lead impedance value, as defined by lying outside the allowable impedance range.

6. The method of at least one of the preceding claims, wherein the threshold number of test data sets is lower than the number of test data sets contained in the observation window, thereby defining an enable window, the enable window being shorter than the observation window and extending backwards in time from the most recent test data set.

7. The method of claim 6, further comprising setting the MRI mode status to a disabled state, if at least one of the test data sets within the enable window has an out-of-range lead impedance value, as defined by lying outside the allowable impedance range.

8. The method of claim 6 or 7, wherein, if there is a gap in the time series through absence of a test data set for a particular time interval, then the counted number is not incremented for the gap and the enable window is extended backwards in time by one time interval.

9. The method of claim 6, 7 or 8, wherein the enable window is adjustable responsive to an analysis of trends in the test data sets that occur over a time period greater than the observation window.

10. The method of any preceding claim, wherein the observation window is adjustable responsive to an analysis of trends in the test data sets that occur over a time period greater than the observation window.

11. The method of any preceding claim, wherein the allowable impedance range is defined by upper and lower impedance values, at least one of which is adjustable responsive to an analysis of trends in the test data sets that occur over a time period greater than the observation window.

12. A computer program product comprising a computer-readable storage medium having program code stored therein that is configured to allow a computer processor to perform the method of any one of claims 1 to 11.

13. A neurostimulation device comprising a computer-readable storage medium and a processor, the storage medium having program code stored therein and the processor being operable to execute the program code to perform the method of any one of claims 1 to 11.

14. A hand-held computing device comprising a computer-readable storage medium loaded with a therapy program, a processor and a wireless transceiver configured to communicate with a neurostimulation device, the storage medium having program code stored therein and the processor being operable to execute the therapy program and the program code to perform the method of any one of claims 1 to 11.

15. A data center comprising a computer-readable storage medium, a processor and a communication transceiver to enable data communication within a medical device communication system, the storage medium having program code stored therein and the processor being operable to execute the program code to perform the method of any one of claims 1 to 11.
